# EUROPEAN PATENT APPLICATION

(11) **EP 4 431 518 A1**
(43) Date of publication of application: **18.09.2024**
(21) Application number: 23802663.7
(22) Date of filing: 26.04.2023
(51) Int. Cl.: C07H 13/04, C07H 1/06

(54) **NEW CRYSTAL FORM OF TRISACCHARIDE**

(30) Priority: 07.05.2022 CN 202210493141
(71) Applicant: Shandong Henglu Biotech. Co., Ltd., Jinan, Shandong 250004 (CN)
(72) Inventor: FANG, Xu, Jinan, Shandong 250004 (CN); YIN, Wencheng, Jinan, Shandong 250004 (CN); JIANG, Fujiao, Jinan, Shandong 250004 (CN); ZHU, Changxiong, Jinan, Shandong 250004 (CN)
(74) Representative: Herrmann, Uwe
(86) International application number: PCT/CN2023/090966
(87) International publication number: WO 2023/216890

(57) **Abstract**

The invention provides a lacto-N-triose II crystal form A and a preparation method therefor. The powder X-ray diffraction pattern of the crystal form has characteristic diffraction peaks at diffraction angles (2θ ± 0.2°) of 4.80, 8.32, 9.62, 12.72, 18.67, 19.90, 21.05, and 21.59 degrees. The lacto-N-triose II crystal form A provided by the invention has good moisture absorption resistance and stability, can reduce packaging costs, and prolongs product shelf life.

## Description

This invention claims the benefit of a prior application CN202210493141.6 submitted to the China National Intellectual Property Administration on May 7, 2022, titled "New Crystalline Form of Trisaccharide." The complete disclosures of the aforementioned application are hereby incorporated by reference into this document as part of the present invention.

### TECHNICAL FIELD

The present invention relates to a new crystal variant of lacto-N-triose II and belongs to the field of separation and purification technology.

### BACKGROUND

Human milk oligosaccharides (HMOs), a diverse and functionally distinct group of complex oligosaccharides, are notably prevalent in human milk. Comprising monosaccharides, derivatives such as sialic acid and various other units linked by glycosidic bonds, over 200 unique oligosaccharide structures have been cataloged, enhancing our knowledge over a century.

The historical journey began in 1886 when the Austrian pediatrician and microbiologist, Escherich, observed a correlation between the intestinal bacteria of infants and their digestive health. In 1900, Moro and Tissier independently corroborated that the bacterial flora in the excrement differed significantly between infants fed on breast milk and those on formula, prompting investigations into potential breast milk components influencing infant gut microbiota. In 1926, Schonfeld identified a whey component that stimulated Bifidobacterium bifidum growth, yet the precise nature of this component remained elusive until 1930 when French scientists Polonowski and Lespagnol discovered carbohydrate-like substances in breast milk whey, terming them "gynolactose." By 1954, György validated that oligosaccharides were indeed responsible for the "bifidus factor" promoting Bifidobacterium growth. In the same year, Polonowski and Montreuil utilized two-dimensional paper chromatography to isolate oligosaccharides from gynolactose. In 1983, Egge and his team successfully identified HMOs using fast atom bombardment mass spectrometry. By 1999, Coppa et al. documented that 30%-50% of HMOs remained structurally intact in the feces of breastfed babies. By the year 2000, it became evident that HMOs resist enzymatic degradation within the infant gut, underscoring their role in biological functions beyond mere nutrition and energy.

The dawn of the 21st century, researches with enhanced methodologies have revealed a decline in the total concentration of HMOs, both acidic and neutral, as lactation progresses. Acknowledging the paramount importance of HMOs for infant development and long-term health, they are known to foster a balanced gut microecology, stimulate beneficial bacterial growth, curb pathogens, resist infectious diseases, diminish the risk of inflammatory bowel conditions and gastroenteritis, modulate the immune response, and support cognitive development in infants. Therefore, timely supplementation of HMOs proves advantageous for the healthy growth of infants and toddlers. The synthesis of sugars has historically posed a significant challenge to synthetic chemists. Unlike the replication of a single template, the construction of sugar chains in living organisms is intricately regulated by multipe glycosyltransferases and glycosidases. This regulatory intricacy begets the structural complexity, variety, and subtle heterogeneity inherent in sugar chains. However, the field of glycochemistry and recombinant enzymology has witnessed rapid advancements, enabling glycochemists and glycobiologists to invest substantial efforts in sugar synthesis. This progress has resulted in a series of innovative methods and strategies for oligosaccharide synthesis being reported.

Lacto-N-triose II (LNTII), also known by its systematic name GlcNAc-β1,3-Gal-β1,4-Glc and identified with the CAS number 75645-27-1, has a chemical structure illustrated in Formula 1.

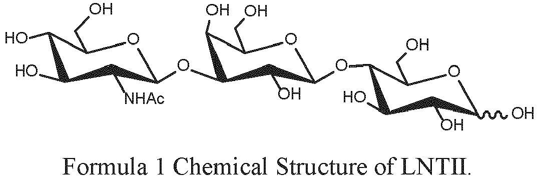

As a key precursor to the backbone of HMOs, LNTII is typically prepared via chemical or biological methods. According to existing literature, its solid form can be attained through methods such as evaporation concentration with subsequent filtration drying, lyophilization, or spray drying techniques. The Chinese invention patent CN112154150A details the enzymatic fermentation synthesis of HMOs and subsequent processing involving enzymes, ultrafiltration, nanofiltration, and chromatographic separation to yield solid oligosaccharides. Notably, this patent comprehensively delineates approaches to solidification without mentioning crystallization for purification. Experimental evidence suggests that this method produces amorphous solids. Another patent, CN109705175A, outlines a purification process for neutral HMOs from a crude solution, incorporating simulated moving bed chromatography and various purification steps like concentration, dialysis, and/or filtration, concluding with the production of an amorphous powder via spray drying, yielding particles ranging from 5 to 500 microns. Patent CN104428307A offers a protocol to substantially reduce or eliminate organic solvent residues in HMOs by spray drying an aqueous HMO solution to achieve amorphous solids.

Despite its utility, the spray drying technique may suffer from material loss due to the adhesion of raw materials to the equipment's inner walls during processing. In comparison to the crystallization method for separation and purification, spray drying is less efficient and more energy-intensive, requiring significant manual input and often resulting in lower purification levels. Moreover, the high-temperature conditions during spray drying can alter the physicochemical properties of certain raw materials. The resulting amorphous powder is highly hygroscopic, complicating storage and handling.

Patent CN110483652A describes a method for obtaining HMOs through lyophilization. However, the amorphous powder obtained requires stringent storage conditions such as freezing or refrigeration, indicating increased preservation costs and complicated handling. Additionally, lyophilization is known for its considerable equipment overhead, high energy consumption, and relatively low yields.

Therefore, to supply the market with a stable, cost-effective solid form of LNTII and to meet the evolving needs of product development, there is an urgent need to devise an efficient LNTII separation technology suitable for on an industrial-scale mass production. This will ensure a superior solid form of LNTII, facilitating storage and expediting its incorporation into dairy products, foods, pharmaceuticals, cosmetics, feeds, and a multitude of other consumer goods.

### SUMMARY

The objective of this invention is to introduce a stable new crystalline form of LNTII, along with its manufacturing process, to advance the separation technology and solid product form of LNTII. This innovation aims to provide a reliable method for mass production at an industrial scale.

Throughout extensive experiments, the inventors have identified a novel crystalline form of LNTII, designated as LNTII crystalline form A, also referred to as crystalline form A within the context of this invention. Its characteristics are as follows:
The technical solution for the invention:
The invention is defined by LNTII crystalline form A, characterized by a powder X-ray diffraction (XRD) spectrum with distinct peaks at 2θ values (2θ ± 0.2°) of 4.80, 8.32, 9.62, 12.72, 18.67, 19.90, 21.05, and 21.59.

In a preferred embodiment, LNTII crystalline form A exhibits a powder XRD spectrum with peaks at 2θ values (2θ ± 0.2°) of 4.80, 7.24, 8.32, 9.62, 12.72, 16.67, 18.67, 19.28, 19.90, 21.05, 21.59, 22.14, and 26.93.

In another preferred embodiment, crystalline form A is characterized by a powder XRD spectrum with peaks at 2θ values (2θ ± 0.2°) of 4.80, 7.24, 8.32, 9.62, 12.72, 16.67, 17.35, 18.67, 19.28, 19.90, 20.72, 21.05, 21.59, 22.14, 23.17, 23.71, 24.15, 25.20, 26.93, 29.12, and 29.49.

In yet another preferred embodiment, crystalline form A presents a powder XRD spectrum with peaks at 2θ values (2θ ± 0.2°) of 4.80, 7.24, 8.32, 9.62, 9.89, 11.65, 12.72, 14.42, 16.67, 17.35, 18.67, 19.28, 19.90, 20.72, 21.05, 21.59, 22.14, 23.17, 23.71, 24.15, 25.20, 26.93, 28.68, 29.12, 29.49, 30.34, 31.08, 31.88, and 34.10.

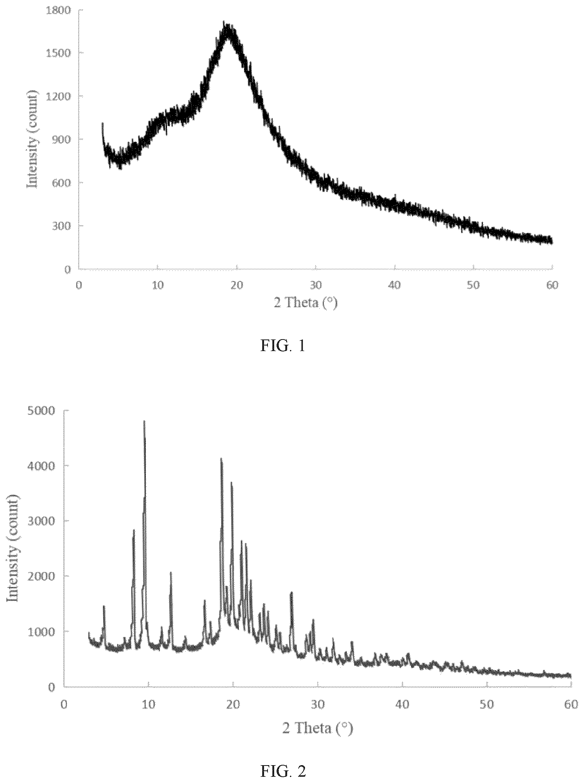

In a further preferred embodiment, crystalline form A is illustrated by the powder XRD chart shown in FIG. 2.

The melting point of LNTII crystalline form A is determined to be within the range of 191-196°C. This contrasts with the amorphous powder form of LNTII mentioned in existing technologies, which has a melting point ranging from 130-134°C.

The method for preparing LNTII crystalline form A entails the following refined steps:
Step 1: A solution of LNTII is prepared for subsequent use.

It is optimal for the LNTII solution to have a concentration ranging from 0.2 to 0.5 g/mL.

Step 2: The prepared LNTII solution is then cooled, ideally to 25°C or lower. For optimal results, it is recommended that the cooling process proceed at a controlled rate of 0.5°C per minute to prevent any abrupt temperature drops, setting the solution aside for later use.

Step 3: While stirring, acetone is introduced to the cooled solution from Step 2 to initiate crystallization.

The LNTII solution can be produced by either dissolving solid LNTII or by refining and concentrating the fermentation broth from a LNTII-producing strain. The strain has been verified for its capacity to produce LNTII via fermentation. The corresponding cultivation technique is also well-established in the field, contingent upon its effectiveness in fermenting the strain to produce LNTII.

A precise volume of acetone is added, with the total being equal to or exceeding the solution's water volume. A preferred ratio is between 1:2 and 1:3 of water to acetone, with an even more preferred range of 1:2.4 to 1:2.7, ideally at 1:2.5.

It is important to note that, acetone is added incrementally, at a rate of 0.07 to 0.25 mL/min, and stirring is maintained post-addition to promote crystal growth. Rapid addition of acetone may compromise crystal structure or purity.

Additionally, the process may incorporate the use of seed crystals to facilitate crystallization.

It is advised that the stirring speed should exceed 100 r/min per during crystallization.

After the acetone has been fully integrated, stirring should be continued for at least 12 hours to allow for adequate crystal development.

The preparation method involves the extraction of LNTII crystalline form A from the LNTII-containing solution, where the solvent is a mixture of water and acetone.

Preferably, LNTII crystalline form A is produced by cooling of a purified fermentation broth containing LNTII to induce crystallization. The fermentation broth has undergone decolorization and desalination before the integration of acetone. Alternatively, the crystalline form A can be derived by dissolving solid LNTII in water to create an aqueous solution, adding acetone, and then crystallizing by cooling. The term "cooling crystallization" means the process of reducing the temperature to below 25°C to achieve crystallization.

LNTII crystalline form A is versatile, serving as an intermediary in synthesizing other sugar forms or compounds, or as an end product, such as a nutritional additive in dairy products or other foods.

### Advantages

This invention introduces a stable LNTII crystalline form A, along with an efficient preparation method, thereby replacing existing technologies for LNTII production and providing reliable techniques for large-scale manufacturing. The crystalline form A presents several following advantages over amorphous powders:
(1) LNTII crystalline form A outperforms amorphous powders in stability, thereby improving long-term storage of LNTII products and their applicability in food and pharmaceutical industries, potentially reducing packaging costs and extending shelf life.
(2) The preparation method of crystalline form A streamlines the separation of LNTII in industrial applications, leading to a reduction in production expenses and an elevation in operational efficiency.

In summary, this invention has obtained the LNTII crystalline form for the first time, achieved a historic leap in HMO separation technology, and will benefit the public.

### BRIEF DESCRIPTION OF THE DRAWINGS

FIG. 1 shows the XRD pattern of amorphous LNTII powder.
FIG. 2 shows the XRD pattern of LNTII crystalline form A.
FIG. 3 shows the scanning electron microscope (SEM) image of amorphous LNTII powder.
FIG. 4 shows the SEM image of LNTII crystalline form A.
FIG. 5 shows the morphology of amorphous LNTII powder and LNTII crystalline form A before being placed in a constant temperature and humidity chamber (with the amorphous powder on the left and LNTII crystalline form A on the right).
FIG. 6 shows the morphology of amorphous LNTII powder and LNTII crystalline form A after exposure to moisture in a constant temperature and humidity chamber at 25°C, 75% relative humidity (with the amorphous powder on the left and LNTII crystalline form A on the right).

### DETAILED DESCRIPTION

It should be noted that all descriptions and examples provided herein serve as illustrations to further elucidate this application. Unless specified otherwise, all technical and scientific terms used herein carry the same meanings as understood by those with ordinary skill in the relevant fields.

The inventive concepts are further clarified with the following examples, which should not be construed as limiting the invention. These examples are solely for illustrative purposes and do not define the full scope of the invention. Experimental procedures in the subsequent examples are performed under standard conditions, unless specific conditions are mentioned.

In this invention, the quantification of LNTII is performed using high-performance liquid chromatography (HPLC) under the conditions listed below:
Chromatographic column: Asahipak NH2P-504E, 4.6 mm × 250 mm, 5 µm particle size; mobile phase composition: acetonitrile to water in a ratio of 65:35; flow rate: 1 mL/min; detection method: UV-VIS detector; wavelength: 210 nm; column temperature: 30°C; injection volume: 10 µL; total run time: 30 minutes.

When referencing the solid form of LNTII in the examples, it pertains to the form recognized within the industry, which can be synthesized using the established methods found in the public domain. For instance, one might employ spray drying techniques to achieve a powdered form or lyophilization for a freeze-dried variant. The scope of the invention encompasses various methodologies for producing the solid forms of LNTII, not limited to those described herein. The LNTII fermentation broth is produced using existing technologies, such as the method outlined in patent CN201680052611.8, entitled "Production of HMOs in a Microbial Host with Modified Input/Output," or as described in the Chinese patent application CN202210493141.6. The content of these documents is hereby incorporated by reference into this disclosure and is considered part of the invention. It is important to note that the invention is not confined to specific LNTII-producing strains but includes any capable of LNTII production through fermentation. The methods for cultivating LNTII-producing strains are well within the capabilities of those skilled in the field to implement and perform, and the invention's scope extends to any fermentation culture method that produces LNTII.

For example, LNTII-producing strains may be prepared by following Example 1 of CN108026556A, which involves the following steps:
*E. coli* BL21(DE3) is selected as the host cell. Metabolic modification involves precise deletion of endogenous gene and introduction of foreign genes into the host genome. The deletions are performed using the method detailed by Datsenko and Wanner (Proc.Nat1.Acad.Sci.USA 97:6640-6645(2000)). The genes to be deleted encode N-acetylglucosamine-6-phosphate deacetylase (nagA) and glucosamine-6-phosphate deaminase (nagB) from the *E. coli* BL21(DE3) genome to inhibit the intracellular breakdown of N-acetylglucosamine. The insertion of non-native genes is accomplished through the EZ-Tn5 transposition technique. Specifically, the gene for N-acetylglucosaminyltransferase 1gtA (bearing the accession number NP_274923) from Neisseria meningitidis MC58, which has undergone codon optimization, is incorporated into the *E. coli* genome.

The culture of the LNTII-producing strain and synthesis of the LNTII are described as follows:
The initial phase involves cell growth:
Cells are grown in a medium with glucose as the carbon source to increase biomass and enzyme levels. Growth continues until the cell growth reaches the late log or stationary phase. Some cells are plated on a solid medium and incubated at 30°C for 2-3 days. Single colonies are selected and transferred to 1.5 mL of liquid medium and incubated at 30°C with shaking at 200 rpm until an OD600 is reached 1.0. Inoculation it into a 5 L liquid medium within a 10 L fermentation vessel follows at a 2% inoculum volume, and incubation occurs overnight at 30°C and 200 rpm. Further inoculation into 50 mL liquid medium flasks at a 2% volume takes place, and incubation with shaking at 30°C and 200 rpm enhances biomass production.

The subsequent phase is to product synthesis: the culture is agitated at 30°C and 200 rpm for 40 hours. Following that, a supplement is continuously introduced until the total fermentation time reaches 72 hours. Post-fermentation, the broth is subjected to centrifugation to separate the cells. The separated cells are lysed using a high-pressure homogenizer. The remaining proteins (if any) are removed by another centrifugation. Finally, the fermentation liquid is combined with the culture supernatant, yielding a fermentation product containing LNTII.

The composition of culture media used is detailed as follows:
Solid medium: 20 g/L peptone, 10 g/L yeast extract, 20 g/L glucose, and 20 g/L agar powder, sterilized at 115°C for 30 minutes.
Liquid medium: 20 g/L peptone, 10 g/L yeast extract, 20 g/L glucose, 8 g/L lactose, and final concentrations of 5 mM each of dipotassium phosphate, magnesium sulfate, ammonium sulfate, and manganese sulfate, sterilized at 115°C for 30 minutes.
Supplements: 2 g/L lactose, 20 g/L glucose, and final concentrations of 5 mM each of dipotassium phosphate, magnesium sulfate, ammonium sulfate, and manganese sulfate.

The preparation of LNTII concentrate and amorphous LNTII powder:
The fermentation product containing LNTII undergoes a series of purification steps involving both anion and cation exchange resins for decolorization and desalination, respectively. Subsequent chromatographic resin purification yields a highly purified concentrate. This concentrate is then further processed to achieve a final LNTII concentration of 466 g/L, thus producing the LNTII concentrate. A portion of this concentrate is reserved, and the remainder is subjected to spray drying to obtain amorphous LNTII powder. XRD analysis validates the amorphous nature of the powder, while HPLC confirms its LNTII content to be 91.24% and identifies a melting point to be 132.3°C.

The LNTII concentrate and amorphous powder obtained above serve as the foundational materials for the subsequent examples.

Example 1: Weigh out 5 g of amorphous LNTII powder. Dissole by mixing with 10 ml of purified water and heating to 50°C until completely dissolved. Once dissolved, cool the solution to 25°C at a rate of 0.5°C/min. Add 0.06 g of seed crystals. Gradually add 30 ml of acetone at a flow rate of 0.25 ml/min while stirring. Allow the crystals to form over a 12-hour period. Collect the solid by filtration, rinse with acetone chilled to 0°C, and dry at 55°C to yield LNTII crystals designated as crystalline form A. The purity of these crystals is determined to be 97.98% by HPLC, with a yield of 90.89%.

Both the spray-dried amorphous LNTII powder before crystallization and the resulted crystalline form are analyzed using XRD, following the methods outlined in the Chinese Pharmacopoeia (2020 edition), Appendix 0451 (second method, powder XRD). The XRD pattern of the amorphous powder is depicted in FIG. 1, confirming its non-crystalline state. The XRD pattern of the crystals from this example is illustrated in FIG. 2, where characteristic peaks are evident. Specific peak positions and intensities are detailed in Table 1.

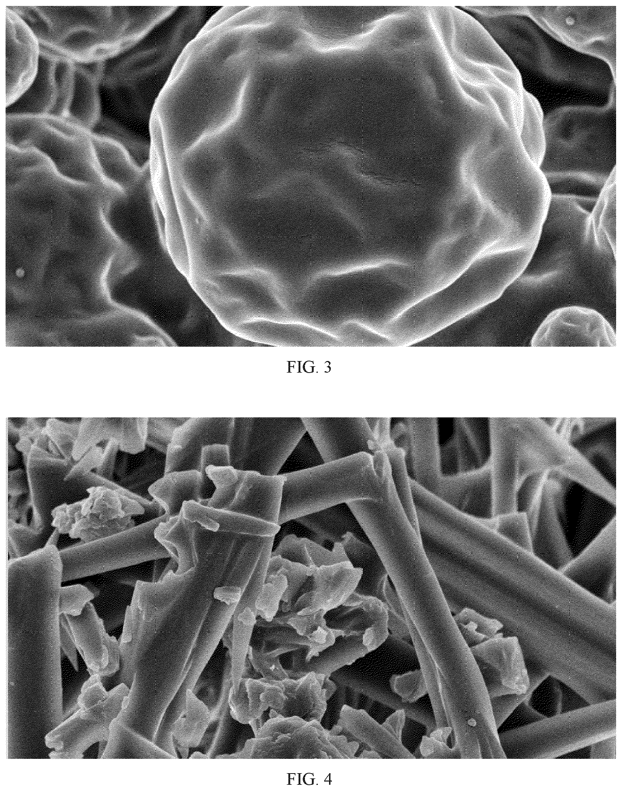

The morphology of both the amorphous LNTII powder before crystallization and crystalline form A obtained through this process are assessed using the JY/T0584-2020 "General Rules for Analytical Methods of SEM." SEM images for the amorphous powder and crystalline form A are provided in FIG. 3 and FIG. 4, respectively.

The melting point of crystalline form A from this example is determined following the GB/T21781-2008 "Test Method for Melting Point and Melting Range of Chemical Products - Capillary Tube Method," and is recorded at 194°C.

**Table 1**

| 2θ° | The relative peak intensity | 2θ | The relative peak intensity |
|---|---|---|---|
| 4.8050 | 587.53 | 29.1229 | 506.39 |
| 7.2426 | 202.17 | 29.4944 | 746.63 |
| 8.3259 | 1946.85 | 30.3452 | 211.02 |
| 9.6166 | 3993.44 | 31.0885 | 229.45 |
| 9.8935 | 437.34 | 31.8784 | 386.04 |
| 11.6522 | 410.28 | 32.6448 | 98.97 |
| 12.7225 | 1403.34 | 33.3669 | 190.95 |
| 14.4217 | 272.85 | 34.1012 | 390.82 |
| 16.6682 | 939.11 | 35.1796 | 93.28 |
| 17.3503 | 589.7 | 36.8714 | 190.66 |
| 18.6696 | 3525.47 | 37.5457 | 187.18 |
| 19.2753 | 1216.09 | 38.2121 | 185.88 |
| 19.9013 | 3128.71 | 40.0989 | 129.7 |
| 20.7155 | 879.6 | 40.6568 | 181.69 |
| 21.0539 | 2030.02 | 41.7371 | 86.97 |
| 21.5864 | 2045.04 | 43.6724 | 126.31 |
| 22.1364 | 1367.75 | 45.1482 | 120.68 |
| 23.1747 | 778.73 | 46.0168 | 86.26 |
| 23.7111 | 965.19 | 47.0811 | 154.57 |
| 24.1495 | 786.48 | 48.5759 | 50.83 |
| 25.2053 | 555.43 | 50.348 | 53.52 |
| 25.6013 | 479.16 | 53.7723 | 23.87 |
| 26.9321 | 1141.14 | 56.8146 | 49.38 |
| 28.6864 | 442.43 | / | / |

Example 2: Begin by weighing 5 g of amorphous LNTII powder and dissolve it in 25 ml of purified water at 40°C until the solution is clear. Next, cool the solution to 15°C at a uniform rate of 0.5°C/min, and introduce 0.02 g of seed crystals. Add 50 ml of acetone at a flow rate of 0.07 ml/min, ensuring constant stirring, and allow the crystal growth to proceed for 20 hours. Afterward, filter the solution, wash the crystals with acetone at 8°C, and dry at 60°C to obtain LNTII crystalline form A. This form is confirmed by an XRD pattern identical to that of Example 1 and has a melting point of 196°C. The purity is 98.18%, and the yield is 92.6%, both ascertained by HPLC.

Example 3: Weigh 5 g of amorphous LNTII powder, add 20 ml of purified water, and dissolve by heating to 55°C until clear. Cool the solution gradually to 10°C at a cooling rate of 0.4°C/min. Then, add 50 ml of acetone at a flow rate of 0.20 ml/min while stirring. Allow the crystals to grow for 48 hours. Filter the product, wash it with acetone cooled to a range of 0-8°C, and dry at 65°C to attain LNTII crystalline form A. This form exhibits an XRD pattern identical to that of Example 1 and has a melting point of 194°C. Its purity and yield, determined via HPLC, are 97.87% and 93.89%, respectively.

Example 4: Take 10 ml of the concentrated LNTII solution with a concentration of 466 g/L, cool it to 25°C. Then, add 0.04 g of seed crystals. Introduce 20 ml of acetone at a flow rate of 0.25ml/min while stirring continuously. Allow the crystal growth to continue undisturbed for 16 hours. Filter the mixture, wash the crystals with acetone cooled to 5°C, and dry at 55°C to obtain LNTII crystalline form A. This form is consistent with the XRD pattern seen in Example 1 and has a melting point of 191°C. Its purity is 92.3% with a yield of 90.89%, as verified by HPLC.

Example 5: Take 10 ml of the concentrated LNTII solution at a concentration of 466 g/L, cool it to 15°C. Then, introduce 0.06 g of seed crystals. Add 30 ml of acetone while stirring at a flow rate of 0.18 ml/min. Allow the crystals to form undisturbed for 16 hours. After the growth period, filter, wash with chilled acetone at 0°C, and dry at 60°C to yield LNTII crystalline form A. This form is characterized by an XRD pattern that matches that of Example 1 and melts at 193°C. Its purity is 92.57%, with a yield of 92.6%, both determined using HPLC.

Example 6: Using the concentrated solution of LNTII at 466 g/L, cool it to 20°C, take 10 ml and introduce 0.06 g of seed crystals. Then, add 25 ml of acetone, stirring at a flow rate of 0.20 ml/min. Allow the crystals to grow undisturbed for 12 hours. Subsequently, filter the crystals, wash with acetone pre-cooled to 0°C, and dry at 65°C to produce LNTII crystalline form A. This form has an XRD pattern identical to that of Example 1, melts at 192°C, and has a purity of 91.98% with a yield of 93.39%, as confirmed by HPLC.

Example 7: Dissolve 5 g of amorphous LNTII powder in 15 ml of purified water at 45°C. After achieving a clear solution, allow it to cool to 25°C before adding 0.06 g of seed crystals. Allow crystal growth for 0.5-1 hour before cooling further to 15°C at a rate of 0.1°C/min. At this temperature, add 40 ml of acetone at a flow rate of 0.15 ml/min with consistent stirring. Allow another 18 hours for crystal growth. Filter the contents, wash with acetone at pre-chilled at 5°C, and dry at 55°C to attain LNTII crystalline form A. Its XRD pattern is identical to that in Example 1, with a melting point of 194°C. Its purity, determined by HPLC, is 98.58%, and the yield is 94.28%.

### Example 8: Hygroscopicity Study

### Step 1: Preparation of glass petri dishes

One day before the experiment, place glass petri dishes (without lids) in a constant temperature and humidity chamber set at 25±1°C and 75±2% relative humidity for 4 hours. Take out the dishes, accurately weigh, and repeat until consistent weight readings are obtained. Record the data in Table 3.

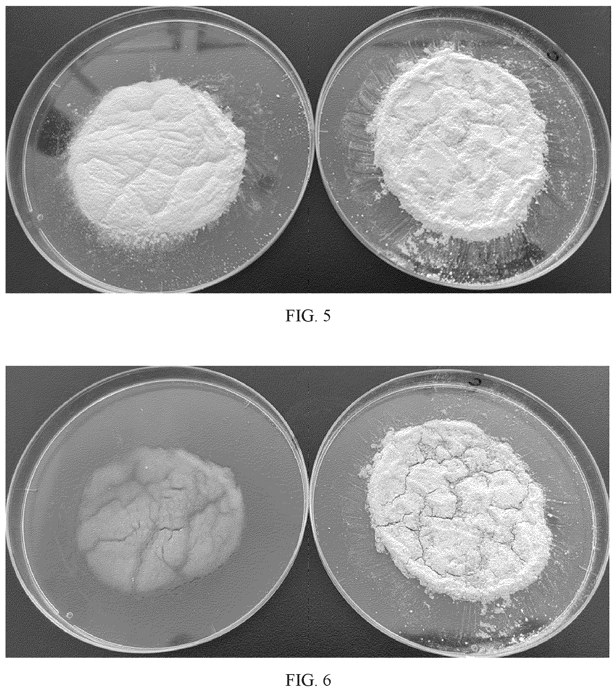

Step 2: Weigh the amorphous LNTII powder and LNTII crystalline form A from Example 1 separately, place them in the pre-weighed petri dishes from Step 1in a constant temperature and humidity chamber set at 25°C and 75% relative humidity. After 5 days, examine any physical changes, reweigh the samples, and calculate their hygroscopicity. Record the results in Tables 2 and 3. A comparison of the physical appearances before and after moisture exposure are depicted in FIG. 5 and FIG. 6.

**Table 2**

| Samples | Before the experiment, appearance | Day 5, appearance |
|---|---|---|
| LNTII amorphous powder | Off- white powder | Light yellow, paste |
| LNTII form A | Off-white | Off-white, no obvious change in appearance |

**Table 3**

| Samples | Before the experiment | | | End of experiment | | |
|---|---|---|---|---|---|---|
| | Weight of culture dish (after constant weight in Step 1), g | Weight of culture dish and sample, g | Weight of sample, g | Weight of culture dish and sample, g | Added weight, g | Moisture rate, % |
| LNTII amorphous | 6.5939 | 7.5951 | 1.0012 | 7.8902 | 0.2951 | 29.47 |
| powder | | | | | | |
| LNTII form A | 6.0407 | 7.0416 | 1.0009 | 7.1834 | 0.1418 | 14.17 |

| | | | | | | |
|---|---|---|---|---|---|---|
| Note: Moisture absorption rate%= (Added weight/sample weight) × 100% | | | | | | |

As indicated by the appearance changes in Table 2 and the hygroscopicity results in Table 3, the stability of LNTII crystalline form A is superior to the amorphous powder, providing benefits for long-term storage and application in food and pharmaceutical industries. This stability has the potential to reduce packaging costs and extend the shelf life of LNTII products.

### Example 9: Hygroscopicity Study

Replicating the procedure outlined in Example 8, evaluate the LNTII crystalline form A samples obtained from Examples 1-7. Place the samples in pre-weighed petri dishes within a constant temperature and humidity chamber at 25°C and 75% relative humidity. After 5 days, inspect for any changes in appearance, weigh the samples, and calculate their hygroscopicity. Document the findings in Table 4.

**Table 4 Hygroscopicity study for LNTII crystalline form A obtained in Example s 1-7**

| | Appearance | Moisture rate, % |
|---|---|---|
| Example 1 Crystalline form A | Off-white, no significant changes | 12.87 |
| Example 2 Crystalline form A | Off-white, no significant changes | 13.26 |
| Example 3 Crystalline form A | Off-white, no significant changes | 14.09 |
| Example 4 Crystalline form A | Off-white, no significant changes | 13.61 |
| Example 5 Crystalline form A | Off-white, no significant changes | 13.79 |
| Example 6 Crystalline form A | Off-white, no significant changes | 13.32 |
| Example 7 Crystalline form A | Off-white, no significant changes | 12.74 |

### Example 10: Stability Study

Separately weigh 10 g of amorphous LNTII powder produced in Examples 1-7. Seal each sample with aluminum-plastic composite film. Store all samples in a constant temperature and humidity chamber at 40°C and 75% relative humidity. Retrieve samples at the end of January, February, and March to assess the LNTII content and observe any changes in appearance. Record the outcomes in Table 5.

**Table 5 Stability study of amorphous LNTII and LNTII crystalline form A**

| | Day 0 | | End of 1 month | | End of 2 months | | End of 3 months | |
|---|---|---|---|---|---|---|---|---|
| | Content, % | Appeara nce | Content, % | Appeara nce | Content, % | Appeara nce | Content, % | Appeara nce |
| Amorph ous LNTII powder | 91.24 | Off-white | 91.01 | Off-white | 90.86 | Off-white | 90.01 | Light yellow |
| Example 1 Crystalli ne form A | 97.98 | Off-white | 98.02 | Off-white | 97.97 | Off-white | 97.96 | Off-white |
| Example 2 Crystalli ne form A | 98.18 | Off-white | 98.15 | Off-white | 98.22 | Off-white | 98.23 | Off-white |
| Example 3 Crystalli ne form A | 97.87 | Off-white | 97.90 | Off-white | 97.93 | Off-white | 97.88 | Off-white |
| Example 4 Crystalli ne form A | 92.30 | Off-white | 92.32 | Off-white | 92.33 | Off-white | 92.27 | Off-white |
| Example 5 Crystalli ne form A | 92.57 | Off-white | 92.61 | Off-white | 92.51 | Off-white | 92.64 | Off-white |
| Example 6 Crystalli ne form A | 91.98 | Off-white | 91.94 | Off-white | 91.84 | Off-white | 91.93 | Off-white |
| Example 7 Crystalli ne form A | 98.58 | Off-white | 98.56 | Off-white | 98.48 | Off-white | 98.56 | Off-white |

Data from Table 5 implies that LNTII crystalline form A maintains its content and appearance throughout the stability study, whereas the amorphous LNTII powder decreases the content and the color gradually fades to pale yellow.

Note that the examples given above are intended solely to illustrate the technical solutions of this invention and should not be construed as limiting its scope. Even though the invention has been detailed through these examples, those skilled in the field may make modifications or equivalent substitutions to these technical solutions without straying from the essence and ambit of the invention.

## Claims

1. A crystalline form A of lacto-N-triose II (LNTII), wherein a powder X-ray diffraction pattern exhibits characteristic peaks at 2θ ± 0.2° values of 4.80, 8.32, 9.62, 12.72, 18.67, 19.90, 21.05, and 21.59.

2. The crystalline form A of LNTII according to claim 1, wherein the powder X-ray diffraction pattern exhibits characteristic peaks at 2θ ± 0.2° values of 4.80, 7.24, 8.32, 9.62, 12.72, 16.67, 18.67, 19.28, 19.90, 21.05, 21.59, 22.14, and 26.93.

3. The crystalline form A of LNTII according to claim 1, wherein the powder X-ray diffraction pattern exhibits characteristic peaks at 2θ ± 0.2° values of 4.80, 7.24, 8.32, 9.62, 12.72, 16.67, 17.35, 18.67, 19.28, 19.90, 20.72, 21.05, 21.59, 22.14, 23.17, 23.71, 24.15, 25.20, 26.93, 29.12, and 29.49.

4. The crystalline form A of LNTII according to claim 1, wherein the powder X-ray diffraction pattern exhibits characteristic peaks at 2θ ± 0.2° values of 4.80, 7.24, 8.32, 9.62, 9.89, 11.65, 12.72, 14.42, 16.67, 17.35, 18.67, 19.28, 19.90, 20.72, 21.05, 21.59, 22.14, 23.17, 23.71, 24.15, 25.20, 26.93, 28.68, 29.12, 29.49, 30.34, 31.08, 31.88, and 34.10.

5. The crystalline form A of LNTII according to any one of claims 1 to 4, wherein having a melting point ranging from 191°C to 196°C.

6. A method for preparing the crystalline form A of LNTII according to any one of claims 1 to 4, comprising mixing a solution containing LNTII with an organic solvent to obtain crystalline form A of LNTII;
preferably, the organic solvent is acetone.

7. The method according to claim 6, wherein the volume of the organic solvent is greater than or equal to the volume of water; preferably, the volume ratio of water to the organic solvent is 1:2.4-2.7, and more preferably 1:2.5.

8. The method according to any one of claims 6 to 7, wherein seed crystals are added to the solution containing LNTII, and the mixture is allowed to crystallize for more than 0.5 hours before being mixed with the organic solvent to obtain crystalline form A of LNTII; preferably, the organic solvent is acetone.

9. The use of the crystalline form A of LNTII according to any one of claims 1 to 4 as an intermediate or final product.
